# EUROPEAN PATENT APPLICATION

(11) **EP 4 755 918 A2**
(43) Date of publication of application: **10.06.2026**
(21) Application number: 26159176.2
(22) Date of filing: 20.08.2020
(51) Int. Cl.: C07K 16/28

(54) **COMBINATION OF A UROLITHIN WITH AN IMMUNOTHERAPY TREATMENT**

(30) Priority: 22.08.2019 GB 201912107
(62) Divisional of application: 20756907.0
(71) Applicant: Amazentis SA, 1015 Lausanne (CH)
(72) Inventor: Rinsch, Christopher, 1015 Lausanne (CH); GRETEN, Florian, 61352 Ban Homburg (DE); DRACHSLER, Moritz, 60318 Frankfurt (DE); ZIEGLER, Pau1, 60528 Frankfurt (DE)
(74) Representative: Abel & Imray LLP

(57) **Abstract**

The invention relates to combinations of urolithins with therapeutic agents, particularly combinations of urolithins with immunotherapy treatments, such as immune checkpoint blockage therapies, such as PD-1 antagonists. The invention also relates to pharmaceutical compositions comprising said combinations, processes of preparing said pharmaceutical compositions and methods of treating diseases with said compositions. In one embodiment, the immune checkpoint blockage therapy is selected from an anti-PD-1 antibody, an anti-PD-L1 antibody, an anti-CTLA4 antibody or a fusion protein.

## Description

The invention relates to combinations of urolithins with therapeutic agents, particularly combinations of urolithins with immunotherapy treatments, such as immune checkpoint blockage therapies, such as PD-1 antagonists. The invention also relates to pharmaceutical compositions comprising said combinations, processes of preparing said pharmaceutical compositions and methods of treating diseases with said compositions.

Urolithins have been proposed as treatments for a variety of conditions related to inadequate mitochondrial activity, including obesity, reduced metabolic rate, metabolic syndrome, diabetes mellitus, cardiovascular disease, hyperlipidaemia, neurodegenerative diseases, cognitive disorders, mood disorders, stress, and anxiety disorders; for weight management, or to increase muscle performance or mental performance. See WO2012/088519 (Amazentis SA). In WO2007/127263 (The Regents of the University of California), the use of urolithins for the treatment of various neoplastic diseases is described.

International patent publication WO2014/004902 (derived from application PCT/US2013/48310) discloses a method of increasing autophagy, including specifically mitophagy, in a cell, comprising contacting a cell with an effective amount of a urolithin or a pharmaceutically acceptable salt thereof, thereby increasing autophagy, including specifically mitophagy, in the cell. Administration may be to a subject having a disease or condition selected from metabolic stress, cardiovascular disease, endothelial cell dysfunction, sarcopenia, muscle degenerative disease, Duchenne muscular dystrophy, alcoholic liver disease, non-alcoholic fatty liver disease, drug-induced liver or muscle injury, α1-antitrypsin deficiency, ischemia/reperfusion injury, inflammation, aging of the skin, inflammatory bowel disease, Crohn's disease, obesity, metabolic syndrome, type II diabetes mellitus, hyperlipidaemia, osteoarthritis, neurodegenerative disease, Alzheimer's disease, Huntington's disease, Parkinson's disease, amyotrophic lateral sclerosis, age-related macular degeneration, mitochondrial diseases (including for example poor growth, loss of muscle coordination, muscle weakness, visual problems, hearing problems, heart disease, liver disease, kidney disease, gastrointestinal disorders, respiratory disorders, neurological problems, autonomic dysfunction sometimes learning disabilities, and dementia (as a result of mitochondrial disease), muscle diseases; cancer, cognitive disorder, stress, and mood disorder.

The immune system is tightly controlled by a network of co-stimulatory and co-inhibitory ligands and receptors. These molecules provide the second signal for T cell activation and provide a balanced network of positive and negative signals to maximize immune responses against infection, while limiting immunity to self (Wang et al. (Epub Mar. 7, 2011) J. Exp. Med. 208(3):577-92; Lepenies et al. (2008) Endocrine, Metabolic & Immune Disorders-Drug Targets 8:279-288). Examples of costimulatory signals include the binding between the B7.1 (CD80) and B7.2 (CD86) ligands of the antigen-presenting cell (APC) and the CD28 and CTLA-4 receptors of the CD4+ T-lymphocyte (Sharpe et al. (2002) Nature Rev. Immunol. 2:116-126; Lindley et al. (2009) Immunol. Rev. 229:307-321). Binding of B7.1 or B7.2 to CD28 stimulates T cell activation, whereas binding of B7.1 or B7.2 to CTLA-4 inhibits such activation (Dong et al. (2003) Immunolog. Res. 28(I):39-48; Greenwald et al. (2005) Ann. Rev. Immunol. 23:515-548). CD28 is constitutively expressed on the surface of T cells (Gross et al. (1992) J. Immunol. 149:380-388), whereas CTLA-4 expression is rapidly up-regulated following T-cell activation (Linsley et al. (1996) Immunity 4:535-543).

Other ligands of the CD28 receptor include a group of related B7 molecules, also known as the "B7 Superfamily" (Coyle et al. (2001) Nature Immunol. 2(3):203-209; Sharpe et al. (2002) Nature Rev. Immunol. 2:116-126; Collins et al. (2005) Genome Biol. 6:223.1-223.7; Korman *et al.* (2007) supra). Several members of the B7 Superfamily are known, including B7.1 (CD80), B7.2 (CD86), the inducible co-stimulator ligand (ICOS-L), the programmed death-1 ligand (PD-L1; B7-H1), the programmed death-2 ligand (PD-L2; B7-DC), B7-H3, B7-H4 and B7-H6 (Collins *et al.* (2005) supra).

The Programmed Death 1 (PD-1) protein is an inhibitory member of the extended CD28/CTLA-4 family of T cell regulators (Okazaki et al. (2002) Curr Opin Immunol 14: 391779-82; Bennett et al. (2003) J. Immunol. 170:711-8). Other members of the CD28 family include CD28, CTLA-4, ICOS and BTLA. PD-1 is suggested to exist as a monomer, lacking the unpaired cysteine residue characteristic of other CD28 family members. PD-1 is expressed on activated B cells, T cells, and monocytes.

The PD-1 gene encodes a 55 kDa type I transmembrane protein (Agata et al. (1996) Int Immunol. 8:765-72). Although structurally similar to CTLA-4, PD-1 lacks the MYPPY motif that is important for B7-1 and B7-2 binding. Two ligands for PD-1 have been identified, PD-L1 (B7-H1) and PD-L2 (B7-DC), that have been shown to downregulate T cell activation upon binding to PD-1 (Freeman et al. (2000) J. Exp. Med. 192:1027-34; Carter et al. (2002) Eur. J. Immunol. 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9).

PD-1 is known as an immunoinhibitory protein that negatively regulates TCR signals (Ishida, Y. et al. (1992) EMBO J. 11:3887-3895; Blank, C. et al. (Epub 2006 Dec. 29) Immunol. Immunother. 56(5):739-745). The interaction between PD-1 and PD-L1 can act as an immune checkpoint, which can lead to, e.g., a decrease in tumour infiltrating lymphocytes, a decrease in T-cell receptor mediated proliferation, and/or immune evasion by cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD- L1 or PD-L2; the effect is additive when the interaction of PD-1 with PD-L2 is blocked as well (Iwai et al. (2002) PNAS USA 99:12293-7; Brown et al. (2003) J. Immunol. 170:1257-66).

Antibody inhibitors of immunological checkpoints, including PD-1 and PD-L1, have demonstrated significant antitumour activity in patients with various solid tumours with less toxicity than broad immune activators, such as IL-2 and IFN-a. Two monoclonal antibodies targeting PD-1, pembrolizumab and nivolumab, have demonstrated significant single agent activity in melanoma, non-small cell lung carcinoma (NSCLC), triple negative breast cancer (TNBC) and other solid tumours (Topalian et al, (2012) N. Engl. J. Med. 366:2443-54; Hamid et al, (2013), N. Engl. J. Med. 369:134-44; Topalian et al, (2014) J. Clin. Oncol. 32:1020-31; Seiwert et al, (2014) J. Clin. Oncol (Meeting Abstracts) 32(15s):6011; Powles et al, (2014) Nature 515:558-62; Garon et al, (2015) N. Engl. J. Med 372:2018-28; Moreno & Ribas (2015) Br. J. Cancer 112:1421-7; Robert et al, (2015) N. Engl. J. Med. 372:320-30). In patients with previously treated, unresectable melanoma the response rates to pembrolizumab and nivolumab were 34% and 31%, respectively; and the progression free survivals were 50 weeks and 9.7 months, respectively (Ribas et al, (2014) J. Clin. Oncol. (Meeting Abstracts) 32(15s):LBA9000; Topalian *et al,* (2014) supra). In patients with advanced, previously untreated non-small cell lung cancer the response rates to pembrolizumab and nivolumab were 26% and 30%, (Rizvi et al, (2014) J. Clin. Oncol. (Meeting Abstracts) 32(15s):8007; Gettinger et al, (2014) J. Clin. Oncol. (Meeting Abstracts) 32(15s):8024). Despite significant activity in some patients, as can be seen from the response rates detailed above, the majority of patients treated with single agent anti-PD-1 immunotherapy do not benefit from treatment.

It is believed that therapeutic approaches that enhance anti-tumour immunity could work more effectively when used in combination with other agents. Surprisingly, we have found that urolithin significantly enhances the efficacy of an immunotherapy treatment, for example, a PD-1 antagonist.

According to a first aspect of the invention there is provided a combination of a urolithin and an immunotherapy treatment for the treatment of a disease state associated with inhibition of T-cell activation.

According to a further aspect of the invention there is provided a combination of a urolithin and an immune checkpoint blockage therapy for the treatment of a disease state associated with inhibition of T-cell activation.

According to a further aspect of the invention there is provided a combination of a urolithin and an immunotherapy treatment for use in the manufacture of a medicament for the treatment of a disease state associated with inhibition of T-cell activation.

According to a further aspect of the invention there is provided a combination of a urolithin and an immune checkpoint blockage therapy for use in the manufacture of a medicament for the treatment of a disease state associated with inhibition of T-cell activation.

According to a further aspect of the invention there is provided a method of treating a disease state associated with inhibition of T-cell activation using a combination of a urolithin and an immunotherapy treatment.

According to a further aspect of the invention there is provided a method of treating a disease state associated with inhibition of T-cell activation using a combination of a urolithin and an immune checkpoint blockage therapy.

According a further aspect of the invention there is provided a combination of a urolithin and an agent which modulates immune-inhibitory proteins, such as PD-1 or PD-L1, for use in the treatment of a disease state associated with inhibition of T-cell activation.

According a further aspect of the invention there is provided a combination of a urolithin and an agent which modulates immune-inhibitory proteins, such as PD-1 or PD-L1 for use in the manufacture of a medicament for the treatment of a disease state associated with inhibition of T-cell activation.

According a further aspect of the invention there is provided a method of treating a disease state associated with inhibition of T-cell activation using a combination of a urolithin and an agent which modulates immune-inhibitory proteins, such as PD-1 or PD-L1.

Compounds, or compounds and treatments, of the invention may be administered by separate, sequential or simultaneous administration.

According a further aspect of the invention there is provided a combination of a urolithin and an immunotherapy treatment for use in therapy.

According a further aspect of the invention there is provided a combination of a urolithin and an immune checkpoint blockage therapy for use in therapy.

According a further aspect of the invention there is provided a combination of a urolithin and an agent which modulates immune-inhibitory proteins for use in therapy.

According a further aspect of the invention there is provided a composition, for example a pharmaceutical composition, comprising a combination of a urolithin and an immunotherapy treatment for use in therapy.

According a further aspect of the invention there is provided a composition, for example a pharmaceutical composition, comprising a combination of a urolithin and an immune checkpoint blockage therapy for use in therapy.

According a further aspect of the invention there is provided a composition, for example a pharmaceutical composition, comprising a combination of a urolithin and an agent which modulates immune-inhibitory proteins for use in therapy.

According a further aspect of the invention there is provided a combination of a urolithin and an immunotherapy treatment.

According a further aspect of the invention there is provided a combination of a urolithin and an immune checkpoint blockage therapy.

According a further aspect of the invention there is provided a combination of a urolithin and an agent which modulates immune-inhibitory proteins.

According a further aspect of the invention there is provided a composition, for example, a pharmaceutical composition, comprising a combination of a urolithin and an immunotherapy treatment.

According a further aspect of the invention there is provided a composition, for example, a pharmaceutical composition, comprising a combination of a urolithin and an immune checkpoint blockage therapy.

According a further aspect of the invention there is provided a composition, for example, a pharmaceutical composition, comprising a combination of a urolithin and an agent which modulates immune-inhibitory proteins.

In one embodiment, the immune checkpoint blockage therapy is selected from: a PD-1 antagonist, an anti-CTLA4 therapy, a CD28 antagonist, a B7 ligand antagonist (for example, an antagonist of B7-1 (CD80) or B7-2 (CD86)), a CD27 antagonist, a CD40 antagonist, a CD40 Ligand, an OX40 antagonist, a GITR antagonist, a CD137 antagonist and/or a 41-BB-1 antagonist.

In one embodiment, the immune checkpoint blockage therapy is selected from: a PD-1 antagonist, an anti-CTLA4 therapy, and a CD28 antagonist, and/or a B7 ligand antagonist (for example, an antagonist of B7-1 (CD80), B7-2 (CD86), a CD27 antagonist, a CD40 antagonist, a CD40 Ligand, an OX40 antagonist, a GITR antagonist, a CD137 antagonist and/or a 41-BB- I antagonist.

In a further embodiment, the PD-1 antagonist is an anti-PD-1 antibody or functional part thereof. Examples of anti-PD-1 antibodies include pembrolizumab, nivolumab (BMS-936558), cemiplimab and pidilizumab.

In a further embodiment, the PD-1 antagonist is an anti-PD-L1 antibody or functional part thereof. Examples of anti-PD-L1 antibodies include avelumab, atezolizumab (MPDL3280A) and durvalumab.

In another embodiment, the PD-1 antagonist is a fusion protein such as AMP-224 (a recombinant B7-DC Fc-fusion protein composed of the extracellular domain of the PD-1 ligand programmed cell death ligand 2 (PD-L2, B7-DC) and the Fc region of human immunoglobulin (Ig) G₁).

In a further embodiment, the immune checkpoint blockage therapy is an anti-CTLA4 therapy. Examples of anti-CTLA4 therapy include: ipilimumab and tremelimumab

The combination of the invention is useful for the treatment of diseases wherein there is a block in processes, which lead to T-cell activation. Examples of such diseases include cancer and infectious diseases.

Examples of suitable cancers include: solid tumours, including HIV-associated metastatic solid tumours.

Examples of suitable cancers include: bladder cancer, B-cell lymphoma such as Hodgkin's lymphoma, T-cell lymphoma, T-cell acute lymphoblastic leukemia, acute lymphoblastic leukemia, chrome lymphoblastic leukemia, acute myelogenous leukemia, chronic myelogenous leukemia, erythroleukemia, triple negative breast cancer, breast cancer, ovarian cancer, melanoma including paediatric melanoma, lung cancer such as squamous cell lung carcinoma and non small-cell lung cancer, pancreatic cancer, glioblastoma, colorectal cancer, head and neck cancer such a head and neck squamous cell carcinoma, cervical cancer, prostate cancer, liver cancer, oral squamous cell carcinoma, skin cancer, medulloblastoma, hepatocellular carcinoma, intrahepatic and extrahepatic cholangiocarcinoma, desmoid tumours, soft tissue sarcoma, adenoid cystic carcinoma, urothelial cancer, renal cancer, hepatocellular cancer, skin cancer, such as Merkel cell carcinoma, gastric cancer and gastroesophageal cancer.

In one embodiment, the cancer is colorectal cancer.

In one embodiment, a suitable cancer is a microsatellite instability high (MSI-H) or mismatch repair deficient (dMMR) solid tumour.

Examples of infectious diseases include: viral, bacterial, fungal and parasitic infection.

Examples of viral infections include: lymphocytic choriomeningitis virus (LCMV), HIV, hepatitis B virus (HBV), or hepatitis C virus (HCV).

Examples of bacterial infections include: Helicobacter pylori, Mycobacterium tuberculosis (MTB), sepsis (gram positive bacteria such as staphylococcus) and nosocomial (hospital acquired infections, such as C. difficile).

Examples of parasitic infections include: helminth parasites, including Schistosoma mansoni, Schistosoma japonicum, Schistosoma haematobium, Fasciola hepatica, and Heligmosomoides polygyrus, Leishmania such as Leishmania donovani, Leishmania chagasi and Leishmania Mexicana, Plasmodium, such as Plasmodium berghei and Plasmodium falciparum, Toxoplasma, such as Toxoplasma gondii.

Examples of fungal infections include: Candidiasis, Aspergillosis and Cryptococci.

Examples of infectious diseases include, but are not limited to HIV, Hepatitis (A, B, & C), Influenza, Herpes, Giardia, Malaria, Leishmania, Staphylococcus aureus and Pseudomonas Aeruginosa.

### Additional/Combination Therapy

Combinations of the invention may be combined with further therapies, such as radiation therapy and/or one or more therapeutic agents.

In some embodiments, the composition further comprises one or more therapeutic agents comprising, anti-cancer agents, anti-viral agents, anti-inflammatory agents, and or adjuvants.

In one embodiment the further therapy is radiation therapy.

In one mode, the radiation therapy is fractionated radiation therapy. In one embodiment, the fractionated radiation therapy comprises from 2 to 7 fractions. In another embodiment, the fractionated radiation therapy comprises from 3 to 6 fractions. In another embodiment, the fractionated radiation therapy comprises from 4 to 5 fractions. In one mode, the fractionated radiation therapy comprises 2, 3, 4, 5, 6, or 7 fractions. In one embodiment, the fractionated radiation therapy comprises 5 fractions.

In one mode, the radiation therapy fractions are administered in sequential days. In one mode, radiation therapy may include more than one dose on a day and/or doses on sequential days. In one mode, the radiation therapy fractions are administered on day 1, day 2, day 3, day 4, and day 5. In another mode, the radiation therapy comprises about 10 Gy in 5 fractions (i.e., 2 Gy on each of 5 days).

Other fractionation schedules may be employed including accelerated fractionation (treatment given in larger daily or weekly doses to reduce the number of weeks of treatment), hyperfractionation (smaller doses of radiation given more than once a day), or hypofractionation (larger doses given once a day or less often to reduce the number of treatments).

The radiation therapy may be x-rays, gamma rays, or charged particles. The radiation therapy may be external-beam radiation therapy or internal radiation therapy (also called brachytherapy). Systemic radiation therapy, using radioactive substances, such as radioactive iodine, may also be employed.

External-beam radiation therapy includes 3D conformational radiation therapy, intensity-modulated radiation therapy, image-guided radiation therapy, tomotherapy, stereotactic radiosurgery, proton therapy, or other charged particle beams.

In another embodiment, the one or more therapeutic agents, includes, but are not limited to, small molecules, synthetic drugs, peptides (including cyclic peptides), polypeptides, proteins, nucleic acids (e.g., DNA and RNA nucleotides including, but not limited to, antisense nucleotide sequences, triple helices, RNAi, and nucleotide sequences encoding biologically active proteins, polypeptides or peptides), antibodies, synthetic or natural inorganic molecules, mimetic agents, and synthetic or natural organic molecules.

Specific examples of such therapeutic agents include, but are not limited to, immunomodulatory agents (e.g., interferon), anti-inflammatory agents (e.g., adrenocorticoids, corticosteroids (e.g., beclomethasone, budesonide, flunisolide, fluticasone, triamcinolone, methylprednisolone, prednisolone, prednisone, hydrocortisone), glucocorticoids, steroids, and non-steroidal anti- inflammatory drugs (e.g., aspirin, ibuprofen, diclofenac, and COX-2 inhibitors), pain relievers, leukotriene antagonists (e.g., montelukast, methyl xanthines, zafirlukast, and zileuton), beta2-agonists (e.g., albuterol, biterol, fenoterol, isoetharie, metaproterenol, pirbuterol, salbutamol, terbutalin formoterol, salmeterol, and salbutamol terbutaline), anticholinergic agents (e.g., ipratropium bromide and oxitropium bromide), sulphasalazine, penicillamine, dapsone, anti-histamines, anti-malarial agents (e.g., hydroxychloroquine), anti-viral agents (e.g., nucleoside analogs (e.g., remdesivir, zidovudine, acyclovir, ganciclovir, vidarabine, idoxuridine, trifluridine, and ribavirin), foscamet, amantadine, rimantadine, saquinavir, indinavir, ritonavir, and AZT) and antibiotics (e.g., dactinomycin (formerly actinomycin), bleomycin, erythromycin, penicillin, mithramycin, and anthramycin (AMC)).

Any therapy which is known to be useful, or which has been used or is currently being used for for the treatment of a disease state associated with inhibition of T-cell activation can be used with a combination of the invention. See, e.g., Gilman et al, Goodman and Gilman's: The Pharmacological Basis of Therapeutics, 13th ed., McGraw-Hill, New York, 2017; The Merck Manual of Diagnosis and Therapy, Robert S. Porter, M.D. et al. (eds.), 20th Ed., Merck Sharp & Dohme Research Laboratories, Rahway, NJ, 2018; Cecil Textbook of Medicine, 25th Ed., Goldman and Schafer (eds.), Elsevier, 2015, and Physicians' Desk Reference (71st ed. 2016) for information regarding therapies (e.g., prophylactic or therapeutic agents) which have been or are currently being used for the treatment of a disease state associated with inhibition of T-cell activation.

Non-limiting examples of one or more other therapies that can be used in addition to a combination of the invention include immunomodulatory agents, such as but not limited to, chemotherapeutic agents and non-chemotherapeutic immunomodulatory agents. Non-limiting examples of chemotherapeutic agents include methotrexate, cyclosporin A, leflunomide, cisplatin, ifosfamide, taxanes such as taxol and paclitaxol, topoisomerase I inhibitors (e.g., CPT-11, topotecan, 9-AC, and GG-211), gemcitabine, vinorelbine, oxaliplatin, 5-fluorouracil (5-FU), leucovorin, vinorelbine, temodal, cytochalasin B, gramicidin D, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin homologs, and cytoxan.

### Urolithins

Urolithins are metabolites produced by the action of mammalian, including human, gut microbiota on ellagitannins and ellagic acid. Ellagitannins and ellagic acid are compounds commonly found in foods such as pomegranates, nuts and berries. Ellagitannins are minimally absorbed in the gut themselves. Urolithins are a class of compounds with the representative structure (I) shown below. The structures of some particularly common urolithins are described in Table 1 below, with reference to structure (I).

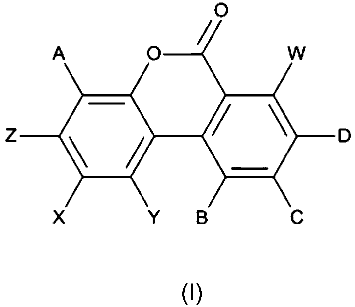

| | Substituent of structure (I) | | | | | |
|---|---|---|---|---|---|---|
| | A | B | C | D | W, X and Y | Z |
| Urolithin A | H | H | H | OH | H | OH |
| Urolithin B | H | H | H | H | H | OH |
| Urolithin C | H | H | OH | OH | H | OH |
| Urolithin D | OH | H | OH | OH | H | OH |
| Urolithin E | OH | OH | H | OH | H | OH |
| Isourolithin A | H | H | OH | H | H | OH |
| Isourolithin B | H | H | OH | H | H | H |
| Urolithin M-5 | OH | OH | OH | OH | H | OH |
| Urolithin M-6 | H | OH | OH | OH | H | OH |
| Urolithin M-7 | H | OH | H | OH | H | OH |

In practice, for commercial scale products, it is convenient to synthesise the urolithins. Routes of synthesis are described, for example, in WO 2014/004902, WO 2015/100213 and WO 2019/168972.

Urolithins of any structure according to structure (I) may be used in the combinations of the invention.

In one aspect of a combinations of the invention, a suitable compound is a compound of formula (I) wherein A, C, D and Z are independently selected from H and OH and B, W, X and Y are all H, preferably at least one of A, C, D and Z is OH.

Particularly suitable compounds are the naturally-occurring urolithins. Thus, Z is preferably OH and W, X and Y are preferably all H. When W, X and Y are all H, and A, and B are both H, and C, D and Z are all OH, then the compound is Urolithin C. When W, X and Y are all H, and A, B and C are all H, and D and Z are both OH, then the compound is urolithin A. Preferably, the urolithin used in the methods of the present disclosure is urolithin A, urolithin B, urolithin C or urolithin D. Most preferably, the urolithin used is urolithin A.

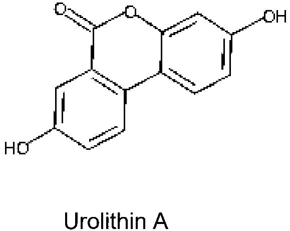

According to one embodiment there is provided a combination of the invention wherein the compound of formula (I) is urolithin A.

According to one embodiment there is provided a combination of the invention wherein the compound of formula (I) is urolithin B.

According to one embodiment there is provided a combination of the invention wherein the compound of formula (I) is urolithin C.

According to one embodiment there is provided a combination of the invention wherein the compound of formula (I) is urolithin D.

In one embodiment, urolithins do not include acylated urolithins or optionally substituted acylated urolithins, (for example, acylated urolithin A, acylated urolithin B, acylated urolithin C, acylated urolithin D, acylated urolithin E, or acylated urolithin M5; orurolithin C having at least one hydroxyl substituted with a group containing a fatty acid). The term "acyl," as used herein, represents a chemical substituent of formula -C(0)-R, where R is alkyl, alkenyl, aryl, arylalkyl, cycloalkyl, heterocyclyl, heterocyclyl alkyl, heteroaryl, or heteroaryl alkyl. An optionally substituted acyl is an acyl that is optionally substituted as described herein for each group R. Examples of acyl include fatty acid acyls (e.g., short chain fatty acid acyls (e.g., acetyl)) and benzoyl.

The present invention also encompasses use of suitable salts of compounds of formula (I), e.g. pharmaceutically acceptable salts. Suitable salts according to the invention include those formed with organic or inorganic bases. Pharmaceutically acceptable base salts include ammonium salts, alkali metal salts, for example those of potassium and sodium, alkaline earth metal salts, for example those of calcium and magnesium, and salts with organic bases, for example dicyclohexylamine, N-methyl-D- glucomine, morpholine, thiomorpholine, piperidine, pyrrolidine, a mono-, di- or tri-lower alkylamine, for example ethyl-, tert-butyl-, diethyl-, diisopropyl-, triethyl-, tributyl- or dimethyl- propylamine, or a mono-, di- or trihydroxy lower alkylamine, for example mono-, di- or triethanolamine.

### Immunotherapy treatment

Immunotherapy treatment for use in combinations of the invention include any treatment whose mechanism of action, in part or predominantly, acts via enhancing an individual's immune response. For example, immune checkpoint blockage therapy, such as an anti-PD-1 antibody, an anti-PD-L1 antibody, an anti-CTLA4 antibody or a fusion protein, neoadjuvant immunotherapy and CAR-T immunotherapy (chimeric antigen receptor T-cell therapy),

### Anti-PD-1 antibodies

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to down regulate T cell activation and cytokine secretion upon binding to PD-1. Inhibition of the PD-1/PD-L1 interaction mediates potent antitumour activity in preclinical models.

Human monoclonal antibodies (HuMAbs) that bind specifically to PD-1 with high affinity have been disclosed in U.S. Patent Nos. 8,008,449 and 8,779,105. Other anti-PD-1 mAbs have been described in, for example, U.S. Patent Nos. 6,808,710, 7,488,802, 8,168,757 and 8,354,509, and PCT Publication No. WO 2012/145493. Each of the anti-PD-1 HuMAbs disclosed in U.S. Patent No. 8,008,449 has been demonstrated to exhibit one or more of the following characteristics:
(a) binds to human PD-1 with a KD of 1 x 10⁻⁷ M or less, as determined by surface plasmon resonance using a Biacore biosensor system;
(b) does not substantially bind to human CD28, CTLA-4 or ICOS;
(c) increases T-cell proliferation in a Mixed Lymphocyte Reaction (MLR) assay;
(d) increases interferon-γ production in an MLR assay;
(e) increases IL-2 secretion in an MLR assay;
(f) binds to human PD-1 and cynomolgus monkey PD-1;
(g) inhibits the binding of PD-L1 and/or PD-L2 to PD-1;
(h) stimulates antigen-specific memory responses;
(i) stimulates antibody responses; and
(j) inhibits tumour cell growth *in vivo.*

Anti-PD-1 antibodies useful for combinations of the present invention include mAbs that bind specifically to human PD-1 and exhibit at least one, at least two, at least three, at least four, or at least five, of the preceding characteristics. In one embodiment, the anti-PD-1 antibody is nivolumab. Nivolumab (also known as "OPDIVO^{®}"; formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumour T-cell functions (U.S. Patent No. 8,008,449; Wang et al., Cancer Immunol Res. 2(9):846-56 (2014)). In another embodiment, the anti-PD-1 antibody or fragment thereof cross-competes with nivolumab. In other embodiments, the anti-PD-1 antibody or fragment thereof binds to the same epitope as nivolumab. In certain embodiments, the anti-PD-1 antibody has the same CDRs as nivolumab.

In another embodiment, the anti-PD-1 antibody or fragment thereof cross-competes with pembrolizumab. In some embodiments, the anti-PD-1 antibody or fragment thereof binds to the same epitope as pembrolizumab. In certain embodiments, the anti-PD-1 antibody has the same CDRs as pembrolizumab. In another embodiment, the anti-PD-1 antibody is pembrolizumab. Pembrolizumab (also known as "KEYTRUDA^{®}", lambrolizumab, and MK-3475) is a humanized monoclonal IgG4 antibody directed against human cell surface receptor PD-1 (programmed death-1 or programmed cell death-1). Pembrolizumab is described, for example, in U.S. Patent Nos. 8,354,509 and 8,900,587; see also http://www.cancer.gov/drugdictionary?cdrid=695789 (last accessed: July 29, 2019). Pembrolizumab has been approved by the FDA for the treatment of relapsed or refractory melanoma. In other embodiments, the anti-PD-1 antibody or fragment thereof cross-competes with MEDI0608. In still other embodiments, the anti-PD-1 antibody or fragment thereof binds to the same epitope as MEDI0608. In certain embodiments, the anti-PD-1 antibody has the same CDRs as MEDI0608. In other embodiments, the anti-PD-1 antibody is MEDI0608 (formerly AMP-514), which is a monoclonal antibody. MEDI0608 is described, for example, in US Pat. No. 8,609,089B2 or in https://www.cancer.gov/publications/dictionaries/cancer-drug/def/anti-pd-1-monoclonal-antibody-medi0680 (last accessed July 29, 2019).

In certain embodiments, the first antibody is an anti-PD-1 antagonist. One example of the anti-PD-1 antagonist is AMP-224, which is a B7-DC Fc fusion protein. AMP-224 is discussed in U.S. Publ. No. 2013/0017199 or in http://www.cancer.gov/publications/dictionaries/cancer-drug?cdrid=700595 (last accessed July 29, 2019).

In other embodiments, the anti-PD-1 antibody or fragment thereof cross-competes with BGB-A317. In some embodiments, the anti-PD-1 antibody or fragment thereof binds the same epitope as BGB-A317. In certain embodiments, the anti-PD-1 antibody has the same CDRs as BGB-A317. In certain embodiments, the anti-PD-1 antibody is BGB-A317, which is a humanized monoclonal antibody. BGB-A317 is described in U.S. Publ. No. 2015/0079109.

In some embodiments, the antibody is Pidilizumab (CT-011), which is an antibody previously reported to bind to PD-1 but which is believed to bind to a different target. Pidilizumab is described in US Pat. No. 8,686,119 B2 or WO 2013/014668 A1.

Anti-PD-1 antibodies useful for combinations of the present invention also include isolated antibodies that bind specifically to human PD-1 and cross-compete for binding to human PD-1 with nivolumab (see, e.g., U.S. Patent Nos. 8,008,449 and 8,779,105; WO 2013/173223). The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar to those of nivolumab by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (see, e.g. WO 2013/173223).

In certain embodiments, the antibodies that cross-compete for binding to human PD-1 with, or bind to the same epitope region of human PD-1 as, nivolumab are mAbs. For administration to human subjects, these cross-competing antibodies can be chimeric antibodies, or humanized or human antibodies. Such chimeric, humanized or human mAbs can be prepared and isolated by methods well known in the art. Anti-PD-1 antibodies useful for the compositions of the disclosed invention also include antigen-binding portions of the above antibodies. It has been amply demonstrated that the antigen-binding function of an antibody can be performed by fragments of a full-length antibody. Examples of binding fragments encompassed within the term "antigen-binding portion" of an antibody include:
(i) a Fab fragment, a monovalent fragment consisting of the V_{L}, V_{H}, C_{L}, and C_{H1} domains;
(ii) a F(ab')2 fragment, a bivalent fragment comprising two Fab fragments linked by a disulfide bridge at the hinge region;
(iii) a Fd fragment consisting of the V_{H} and C_{H1} domains; and
(iv) Fv fragment consisting of the V_{L}, and V_{H} domains of a single arm of an antibody.
Anti-PD-1 antibodies suitable for use in the disclosed compositions are antibodies that bind to PD-1 with high specificity and affinity, block the binding of PD-L1 and/or PD-L2, and inhibit the immunosuppressive effect of the PD-1 signalling pathway. In any of the compositions or methods disclosed herein, an anti-PD-1 antibody includes an antigen-binding portion or fragment that binds to the PD-1 receptor and exhibits the functional properties similar to those of whole antibodies in inhibiting ligand binding and upregulating the immune system. In certain embodiments, the anti-PD-1 antibody or antigen-binding portion thereof cross-competes with nivolumab for binding to human PD-1. In other embodiments, the anti-PD-1 antibody or antigen-binding portion thereof is a chimeric, humanized or human monoclonal antibody or a portion thereof. In certain embodiments, the antibody is a humanized antibody. In other embodiments, the antibody is a human antibody. Antibodies of an IgGI, IgG2, IgG3 or IgG4 isotype can be used. In certain embodiments, the anti-PD-1 antibody or antigen-binding portion thereof comprises a heavy chain constant region which is of a human IgGI or IgG4 isotype. In certain other embodiments, the sequence of the IgG4 heavy chain constant region of the anti-PD-1 antibody or antigen-binding portion thereof contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang et al. Cancer Immunol Res. 2(9):846-56 (2014)). In yet other embodiments, the antibody comprises a light chain constant region which is a human kappa or lambda constant region. In other embodiments, the anti-PD-1 antibody or antigen-binding portion thereof is a mAb or an antigen-binding portion thereof. In certain embodiments of any of the therapeutic methods described herein comprising administration of an anti-PD-1 antibody, the anti-PD-1 antibody is nivolumab. In other embodiments, the anti-PD-1 antibody is pembrolizumab. In other embodiments, the anti-PD-1 antibody is chosen from the human antibodies 17D8, 2D3, 4H1, 4A11, 7D3 and 5F4 described in U.S. Patent No. 8,008,449. In still other embodiments, the anti-PD-1 antibody is MEDI0608 (formerly AMP-514), AMP-224, orBGB-A317. Because anti-PD-1 and anti-PD-L1 target the same signaling pathway and have been shown in clinical trials to exhibit similar levels of efficacy in a variety of cancers, including renal cell carcinoma (RCC) (see Brahmer et al. (2012) N Engl J Med 366:2455-65; Topalian et al. (2012a) NEngl J Med 366:2443-54; WO 2013/173223), an anti-PD-L1 antibody may be substituted for the anti-PD-1 Ab in any of the therapeutic methods disclosed herein. In certain embodiments, the anti-PD-L1 antibody is BMS-936559 (formerly 12A4 or MDX-1105) (see, e.g., U.S. Patent No. 7,943,743; WO 2013/173223). In other embodiments, the anti-PD-L1 antibody is MPDL3280A (also known as RG7446) (see, e.g., Herbst et al. (2013) J Clin Oncol 31(suppl):3000. Abstract; U.S. Patent No. 8,217,149) or MEDI4736 (Khieif (2013) In: Proceedings from the European Cancer Congress 2013; September 27-October 1, 2013; Amsterdam, The Netherlands. Abstract 802). In certain embodiments, the antibodies that cross-compete for binding to human PD-L1 with, or bind to the same epitope region of human PD-L1 as the above-references PD-L1 antibodies are mAbs. For administration to human subjects, these cross-competing antibodies can be chimeric antibodies, or can be humanized or human antibodies. Such chimeric, humanized or human mAbs can be prepared and isolated by methods well known in the art.

### Anti-PD-L1 Antibodies

In certain embodiments, the present application encompasses use of an anti-PD-L1 antibody in lieu of anti-PD-1 antibody. In one embodiment, the anti-PD-L1 antibody inhibits the binding of PD-L1 receptor, i.e., PD-1, with its ligand PD-L1. Anti-PD-L1 antibodies useful for the invention include antibodies engineered starting from antibodies having one or more of the V_{H} and/or V_{L}, sequences disclosed herein, which engineered antibodies can have altered properties from the starting antibodies. An anti-PD-L1 antibody can be engineered by a variety of modifications as described above for the engineering of modified anti-PD-1 antibodies of the invention.

In some embodiments, an anti-PD-L1 antibody useful for the present methods includes mAb 28-8 set forth in International Patent Application number: WO 2016/176503. In other embodiments, an anti-PD-L1 antibody useful for a combination of the invention comprises mAbs 28-1, 28-12, 29-8 and 20-12 (as disclosed in International Patent Application number: WO 2016/176503) or an antigen-binding portion thereof, for example, including Fab, F(ab')2Fd, Fv, and scFv, di-scFv or bi-scFv, and scFv-Fc fragments, diabodies, triabodies, tetrabodies, and isolated CDRs.

### Anti-CTLA-4 antibodies

Anti-CTLA-4 antibodies of the instant invention bind to human CTLA-4 so as to disrupt the interaction of CTLA-4 with a human B7 receptor. Because the interaction of CTLA-4 with B7 transduces a signal leading to inactivation of T-cells bearing the CTLA-4 receptor, disruption of the interaction effectively induces, enhances or prolongs the activation of such T cells, thereby inducing, enhancing or prolonging an immune response.

HuMAbs that bind specifically to CTLA-4 with high affinity have been disclosed in U.S. Patent Nos. 6,984,720 and 7,605,238. Other anti-PD-1 mAbs have been described in, for example, U.S. Patent Nos. 5,977,318, 6,051,227, 6,682,736, and 7,034,121. The anti-PD-1 HuMAbs disclosed in U.S. Patent No. Nos. 6,984,720 and 7,605,238 have been demonstrated to exhibit one or more of the following characteristics:
(a) binds specifically to human CTLA-4 with a binding affinity reflected by an equilibrium association constant (Kₐ) of at least about 10⁷ M⁻¹, or about 10⁹ M⁻¹, or about 10¹⁰ M⁻¹ to 10¹¹ M⁻¹ or higher, as determined by Biacore analysis;
(b) a kinetic association constant (kₐ) of at least about 10³, about 10⁴, or about 10⁵ m⁻¹ s⁻¹;
(c) a kinetic disassociation constant (k_{d}) of at least about 10³, about 10⁴, or about 10⁵ m⁻¹ s⁻¹; and
(d) inhibits the binding of CTLA-4 to B7-1 (CD80) and B7-2 (CD86).
Anti-CTLA-4 antibodies useful for the present invention include mAbs that bind specifically to human CTLA-4 and exhibit at least one, at least two, or at least three of the preceding characteristics.

An exemplary clinical anti-CTLA-4 antibody is the human mAb 10D1 (now known as ipilimumab and marketed as YERVOY^{®}) as disclosed in U.S. Patent No. 6,984,720. Ipilimumab is an anti-CTLA-4 antibody for use in the methods disclosed herein. Ipilimumab is a fully human, IgG1 monoclonal antibody that blocks the binding of CTLA-4 to its B7 ligands, thereby stimulating T cell activation and improving overall survival (OS) in patients with advanced melanoma.

Another anti-CTLA-4 antibody useful for the present methods is tremelimumab (also known as CP-675,206). Tremelimumab is human IgG2 monoclonal anti-CTLA-4 antibody. Tremelimumab is described in WO2012/122444, U.S. Publ. No. 2012/263677, or WO 2007/113648 A2.

Anti-CTLA-4 antibodies useful for the disclosed composition also include isolated antibodies that bind specifically to human CTLA-4 and cross-compete for binding to human CTLA-4 with ipilimumab or tremelimumab or bind to the same epitope region of human CTLA-4 as ipilimumab or tremelimumab. In certain embodiments, the antibodies that cross-compete for binding to human CTLA-4 with, or bind to the same epitope region of human CTLA-4 as does ipilimumab or tremelimumab, are antibodies comprising a heavy chain of the human IgG1 isotype. For administration to human subjects, these cross-competing antibodies are chimeric antibodies, or humanized or human antibodies. Useful anti-CTLA-4 antibodies also include antigen-binding portions of the above antibodies such as Fab, F(ab')₂, Fd or Fv fragments.

### Urolithin Administration/Dosage Regimes

The combinations of the present disclosure involve oral administration of a urolithin of formula (I) or salt thereof to a subject in a daily amount in the range of 1.7 to 6.0 mmol per day, for example, from 1.7 to 2.7 mmol per day, or from 2.8 to 6.0 mmol per day, for a period between 2 to 16 weeks prior to vaccination. As discussed below, administration of is preferred in the range 250mg to 1000mg urolithin A (which corresponds to about 1.1 to 4.4 mmol) results in a surprisingly good pharmacokinetic profile, compared with a much higher dosage of 2000mg. In one embodiment the dose is 250mg/day, in an alternative embodiment the dose is 500mg/day and in another embodiment the dose is 1000mg/day.

In a further embodiment, administration doses are selected from:
- 250mg once or twice a day;
- 500mg once or twice a day;
- 750mg once or twice a day;
- 1000mg once or twice a day;
- 1250mg once or twice a day; or
- 1500mg once or twice a day

The methods of the present disclosure involve daily administration of the compound of formula (I) or salt thereof, or of a composition containing the compound or salt. In some embodiments the compound or composition is administered once per day, i.e. the compound or composition is to be administered at least once per 24 hour period. In other embodiments the compound, or composition comprising the compound, is administered multiple times per day, for example twice per day, or three or four times per day. In such cases, the daily dosage is divided between those multiple doses. In one embodiment administration is once a day, in a second embodiment administration is twice a day, in a third embodiment administration is three times a day.

The methods of the present disclosure would usually require daily administration of the compound of formula (I) or salt thereof, or of a composition containing the compound or salt, for a period over several months. In some embodiments, the methods may involve administration of the compound of formula (I), or salt thereof, over for example daily for at least 2 weeks, 3 weeks, 4 weeks, 5 weeks, 6 weeks, 8 weeks, 12 weeks, 4 months, 6 months, or for at least a year. In some embodiments, the method comprises administering the compound or salt thereof daily for a period of up to 3 months, up to 6 months, up to 1 year, up to 2 years or up to 5 years. In some embodiments, the method comprises administering the compound or salt daily for a period in the range of from 21 days to 5 years, from 21 days to 2 years, from 21 days to 1 year, from 21 days to 6 months, from 21 days to 12 weeks, from 28 days to 5 years, from 28 days to 2 years, from 28 days to 1 year, from 28 days to 6 months, from 28 days to 4 months, from 28 days to 12 weeks, 6 weeks to 2 years, from 6 weeks to 1 year, from 8 weeks to 1 year, or from 8 weeks to 6 months.

The methods of the present disclosure require daily administration of an amount of compound of formula (I) or salt thereof, of from 0.7 mmol per day up to 2.7 mmol per day thereof or from 0.7 mmol twice per day up to 2.7 mmol twice a day. **In** some embodiments, the amount administered is in the range of from 2.0 to 2.5 mmol. **In** some embodiments, the amount administered is approximately, 1.1, 1.2, 1.3, 1.4. 1.5, 1.6 1.7, 1.8, 1.9, 2.0, 2.1, 2.2, 2.3, 2.4, 2.5, 2.6, or 2.7 mmol. In other embodiments, the amount administered is approximately, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, or 6.0 mmol. In some preferred embodiments the method involves administration of approximately 2.2 mmol per day or 2.2 mmol twice per day of the compound of formula (I) or salt thereof (e.g. of urolithin A). The exact weight of compound that is administered depends on the molecular weight of the compound that is used. For example, urolithin A has a molecular weight of 228g/mol (such that 2.20mmol is 501.6mg) and urolithin B has a molecular weight of 212g/mol (such that 2.20mmol is 466.4mg).

In a further embodiment, the methods of the present disclosure require daily administration of an amount of compound of formula (I) or salt thereof, of from 2.8 mmol per day up to 6.0 mmol per day or twice per day thereof. In some embodiments, the amount administered is in the range of from 4.0 to 4.8 mmol. In some embodiments, the amount administered is approximately, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, or 6.0 mmol. In some preferred embodiments the method involves administration of approximately 4.4 mmol per day or twice per day of the compound of formula (I) or salt thereof (e.g. of urolithin A). The exact weight of compound that is administered depends on the molecular weight of the compound that is used. For example, urolithin A has a molecular weight of 228g/mol (such that 4.40mmol is 1003.2mg) and urolithin B has a molecular weight of 212g/mol (such that 4.40mmol is 932.8mg).

In some embodiments the methods involve administration of urolithin A in an amount in the range of from 400 to 600 mg/day or 400 to 600 mg twice per day. In a preferred embodiment the method involves administration of urolithin A in an amount in the range of from 450 to 550 mg, more preferably approximately 500 mg per day or twice per day.

In other embodiments the methods involve administration of urolithin A in an amount in the range of from 700 to 1300 mg/day twice per day, or in the range of from 750 to 1250 mg, or in the range of from 800 to 1200 mg, or in the range of from 850 to 1150 mg, or in the range of from 900 to 1100 mg per day or twice per day. In a preferred embodiment the method involves administration of urolithin A in an amount in the range of from 950 to 1150 mg/day or twice per day, more preferably approximately 1000 mg/day or twice per day.

In some preferred embodiments, the methods involve administering urolithin A to the subject in an amount in the range of from 4.5 to 11 mg/kg/day, such as 4.5 to 8.5 mg/kg/day. In another embodiment, the methods involve administering urolithin A to the subject in an amount in the range of 5 to 9 mg/kg/day. In another embodiment, the methods involve administering urolithin A to the subject in an amount in the range of from 6.0 to 8 mg/kg/day.

In other preferred embodiments, the methods involve administering urolithin A to the subject in an amount in the range of from 9 to 18 mg/kg/day such as 9 to 17 mg/kg/day. In another embodiment, the methods involve administering urolithin A to the subject in an amount in the range of from 10 to 17 mg/kg/day. In another embodiment, the methods involve administering urolithin A to the subject in an amount in the range of from 11 to 16 mg/kg/day.

Dosage regimes which combine a 500mg dose and a 1000mg dose may be advantageous. For example, a twice daily dosage regime which combines a first dose of 1000mg and a second dose several hours later of 500mg. Said 500mg dose may be 6-18 hours after the 1000mg dose, for example 8-12 hours after the 1000mg dose. For example, about 12 hours after the 1000mg dose. Thus, according to a further aspect of the invention there is provided the treatment of a disease with a compound of Formula (I) which comprises a twice daily dosage regime comprising a first dose of 1000mg, followed by a second dose of 500mg wherein the two doses are separated by 6-18 hours.

The compound of formula (I) or salt thereof, or composition containing the compound of salt, may be administered at any suitable time, for example it may be administered in the morning after sleep or in the evening. In some embodiments it may be preferable for the method to be performed at approximately the same time(s) each day, for example within 15, 30, 60 or 120 minutes of a given time point.

### Immunotherapy Administration/Dosage Regimes

The appropriate dose of an immunotherapy treatment is chosen based on clinical indications by a treating physician. Such treatment may comprise small molecule compounds or macromolecules such as antibodies.

For example, antibodies or functional parts thereof are administered in therapeutically effective amounts. Generally, a therapeutically effective amount may vary with the subject's age, condition, and sex, as well as the severity of the medical condition of the subject. A therapeutically effective amount of an antibody or functional part thereof ranges from about 0.001 to about 30 mg/kg body weight, from about 0.01 to about 25 mg/kg body weight, from about 0.1 to about 20 mg/kg body weight, or from about 1 to about 10 mg/kg. The dosage may be adjusted, as necessary, to suit observed effects of the treatment.

In certain embodiments, antibodies or functional parts thereof are administered antibodies is individually administered at a dosage of at least about 0.1, at least about 0.3, at least about 0.5, at least about 1, at least about 3, at least about 5, at least about 10 or at least about 20 mg/kg, e.g., at least about 1 to at least about 10 mg/kg, e.g, at least about 1 to at least about 3 mg/kg, e.g, at least about 3 mg/kg, e.g, at least about 1 mg/kg. Antibodies or functional parts thereof can be administered at a dosing frequency of at least about once every week, at least about once every 2 weeks, at least about once every 3 weeks, or at least about once every 4 weeks, or at least about once a month, for up to 6 to up to 72 doses, or for as long as clinical benefit is observed, or until unmanageable toxicity or disease progression occurs. In some embodiments, antibodies or functional parts thereof are administered at a dosage of about 1 or about 3 mg/kg. In certain embodiments, the sequenced regimen comprises administering the antibodies or functional parts thereof antibody to the subject at a dosing frequency of once about every week, once about every 2 weeks, once about every 3 weeks, or once about every 4 weeks, or once a month for 6 to 72 doses, or for as long as clinical benefit is observed, or until unmanageable toxicity or disease progression occurs. In other embodiments, the antibodies or functional parts thereof are administered at a dosage of about 1 mg/kg at a dosing frequency of once about every 3 weeks for up to 48 doses.

In one embodiment, the compound of formula (I) is administered daily and the immunotherapy treatment, for example an antibody, is administered every 1 to 4 weeks, such as every 2 to 4 weeks, for example, every 2 or 3 weeks. In general, treatment duration is up to months remission.

Antibodies may be given as a bolus dose, to maximize the circulating levels of antibodies for the greatest length of time after the dose. Continuous infusion may also be used after the bolus dose.

### Compositions

The methods of the present disclosure preferably involve oral administration of the compound of formula (I) or a salt thereof. Any suitable oral composition containing the compound of formula (I) or salt thereof may be used. Accordingly, the use of a range of compositions which contain the compound of formula (I), and which are suitable for oral administration, is envisaged. Thus in some embodiments, the compound of formula (I), or salt thereof, is administered in the form of an oral composition containing the compound of formula (I) or salt thereof and one or more excipients suitable for oral administration. Oral compositions may comprise compositions having the form of a pill, tablet, capsule, caplet, lozenge, pastille, granules, powder for suspension, oral solution, oral suspension, oral emulsion, syrup, or the like.

In a further embodiment of the invention, a compound of formula (I) is administered by any means known to the skilled person for administration of pharmaceutical such as, intramuscular, sublingual, cutaneous, inhalation, ocular and auricular.

Compositions containing the compound of formula (I) may take any physical form suitable for the intended application, for example, they may be in the form of a solid (for example, a tablet or capsule), a semi-solid (for example, a softgel), or a liquid (including emulsions). In some instances, the composition may be in the form of a viscous fluid or a paste. Semi-solid forms may likewise contain excipients conventional in the art. The excipients can, for example, provide a desired hardness, shelf-life and flavour such that the composition has an acceptable taste, an attractive appearance and good storage stability. Semi-solid forms can be in the form of a paste. Where the composition is a softgel, it may for example be provided in a capsule having a shell. The shell may be of a conventional type, for example it may be a soft gelatin-based shell. By way of example, the composition may also be provided inside a hard capsule type of shell. Liquid compositions may be in the form of a medicine, a dietary supplement, or a beverage, each for oral consumption. Liquid formulations may be solutions, emulsions, slurries or other semi-liquids. Excipients in a liquid composition can, for example, provide a shelf-life, visual appearance, flavour and mouth-feel such that the composition has an acceptable taste, an attractive appearance and good storage stability. At certain levels of dilution, a drink may need to be shaken before the subject drinks it, so as to maintain an even suspension of the active ingredient.

In some preferred embodiments, the method comprises administration of a compound of formula (I) or salt thereof (e.g. urolithin A), in micronized form. Micronization enables the compound of formula (I) to disperse or dissolve more rapidly. Micronisation can be achieved by methods established in the art, for example compressive force milling, hamermilling, universal or pin milling, or jet milling (for example spiral jet milling or fluidised-bed jet milling) may be used. Jet milling is especially suitable. If micronized compound is used, then preferably the compound has a D₅₀ size of under 100 µm - that is to say that 50% of the compound by mass has a particle diameter size of under 100 µm. More preferably, the compound has a D₅₀ size of under 75 µm, for example under 50 µm, for example under 25 µm, for example under 20 µm, for example under 10 µm. More preferably, the compound has a D₅₀ in the range 0.5-50 µm, for example 0.5 to 20 µm, for example 0.5 to 10 µm, for example 1.0 to 10 µm, for example 1.5 to 7.5 µm, for example 2.8 to 5.5 µm. Preferably, the compound has a D₉₀ size of under 100 µm. More preferably, the compound has a D₉₀ size of under 75 µm, for example under 50 µm, for example under 25 µm, for example under 20 µm, for example under 15 µm. The compound preferably has a D₉₀ in the range 5 to 100 µm, for example 5 to 50 µm, for example 5 to 20 µm, for example 7.5 to 15 µm, for example 8.2 to 16.0 µm. Preferably, the compound has a D₁₀ in the range 0.5 - 1.0 µm. Preferably, the compound of formula (I) or salt thereof (e.g. urolithin A) has a D₉₀ in the range 8.2 to 16.0 µm, a D₅₀ in the range 2.8 to 5.5 µm and a D₁₀ in the range 0.5 to 1.0 µm.

In a further embodiment, the compound of formula (I) or salt thereof has a size distribution selected from one of the following:
(i) D₅₀ size in the range 0.5 to 50 µm and a D₉₀ size in the range 5 to 100 µm,
(ii) the compound has a D₉₀ size in the range 8.2 to 16.0 µm, a D₅₀ size in the range 2.8 to 5.5 µm and a D₁₀ size in the range 0.5 to 1.0 µm;
(iii) the compound of Formula (I) has a D₅₀ size in the range 0.5 to 20 µm and a D₉₀ size in the range 5 to 50 µm;
(iv) the compound of Formula (I) has a D₅₀ size under 50 µm and a D₉₀ size under 75µm;
(v) the compound of Formula (I) has a D₅₀ size under 25 µm and a D₉₀ size under 50µm;
(iv) the compound of Formula (I) has a D₅₀ size under 10 µm and a D₉₀ size under 20µm;
(v) the compound of Formula (I) has a D₅₀ size under 10 µm and a D₉₀ size under 15µm; or
(vi) the compound of Formula (I) has a D₅₀ size of 10 µm and a D₉₀ size of 20µm.

### Compositions comprising a urolithin or salt thereof, and a medium chain triglyceride

In some preferred embodiments, the compound of formula (I) or salt thereof (e.g. urolithin A) is administered in the form of a composition comprising: a) a medium-chain triglyceride; and b) the compound of formula (I) or salt thereof. Within those embodiments, preferably the compound of formula (I) (e.g. urolithin A) is in micronized form.

By selecting suitable medium chain triglycerides and excipients, the physical form of the composition can be tailored to the requirements of the product in question. For example, in some embodiments the compositions may be pharmaceutical compositions. In some embodiments the compositions may be nutritional compositions.

In many cases, compositions containing a compound of formula (I) and a medium chain triglyceride have the consistency of a viscous liquid or paste, and can be provided as a single serving supplement to a subject's general diet (for example in a bar, gel, or a softgel capsule, hard capsule, or diluted in a drink); alternatively, it can be provided as a part of or the whole of a meal.

Where the methods of the disclosure involve use of a composition comprising a medium-chain triglyceride, the medium-chain triglyceride typically makes up at least 1% w/w of the composition, for example at least 5% w/w, for example at least 10% w/w, for example at least 15% w/w. The medium-chain triglyceride preferably makes up 20% w/w or more of the composition, for example 25% w/w or more by weight, for example 30% w/w or more by weight of the composition. For example the medium-chain triglyceride may make up 1-40% w/w of the composition, 2-40% w/w of the composition, 5-40% w/w of the composition; 10-40% w/w of the composition; 1-99% w/w of the composition, 5-99% w/w of the composition , 10-99% w/w of the composition, 20-99% w/w of the composition, 5-90% w/w of the composition, 10-90% w/w of the composition, for example 20-90% w/w of the composition, 20-80% w/w of the composition for example, 30-80% w/w of the composition, for example 30-70% w/w of the composition, for example 30-60% w/w of the composition, for example 30-50% w/w of the composition, for example 30-40% w/w of the composition, for example 30-35% w/w of the composition. For example the medium-chain triglyceride may make up 40-70% w/w of the composition, for example 50-70% w/w of the composition, for example, 55-65% w/w of the composition.

In such compositions, the compound of formula (I) typically makes up from 0.1 to 80% w/w of the composition, for example 0.1 to 60% w/w, for example 0.25 to 50% w/w. For example the compound of formula (I) may make up 0.5-50% w/w of the composition. If the composition is provided as a part or the whole of a meal then the compound of formula (I) may for example make up 0.25-5% w/w of the composition, for example, 0.3-3% w/w of the composition. If the composition is provided as a single serving supplement to a subject's general diet, then the urolithin typically makes up from 20 to 80% w/w of the composition, for example 20 to 40% w/w, for example 25 to 35% w/w of the composition. For example the urolithin may make up 26-34% w/w of the composition, for example, 28-33% w/w of the composition; for example, 29-32% w/w of the composition, for example 29-31% w/w of the composition.

**In** such compositions, the weight ratio of the medium-chain triglyceride component to the compound of formula (I) is generally in the range 0.01:1 to 100:1, for example 0.5:1 to 100:1, for example 0.5:1 to 50:1, for example 0.5:1 to 5:1; or, for example, 1:1 to 75:1, for example 1:1 to 50:1, for example 1:1 to 20:1, for example 1:1 to 10:1, for example 1:1 to 2.5:1, for example 1:1 to 2:1, for example 1:1 to 1.5:1. The weight ratio may be in the ratio 0.01:1 to 10:1, for example 0.1:1 to 10:1 or 0.01:1 to 5:1, for example 0.01:1 to 0.1:1.

In some preferred embodiments, the method of the present disclosure involves administration of a softgel capsule comprising a filling, which filling comprises the compound of formula (I) or salt thereof (e.g. urolithin A) and one or more medium-chain triglycerides. Within those embodiments, preferably the compound of formula (I) or salt thereof (e.g. urolithin A) is micronized. In embodiments where a softgel capsule is used, the shell component may be produced using conventional ingredients.

Medium-chain triglycerides are compounds of formula CH₂(OR¹)-CH(OR²)-CH₂(OR³) where R¹, R² and R³ are medium chain fatty acid groups, generally of formula - C(=O)(CH₂)ₙCH₃ where n is in the range 4 to 10, for example 6 to 8. Medium-chain fatty acids are fatty acids which have an aliphatic tail of 6 -12 carbon atoms. The aliphatic tail is predominantly saturated. Particular medium-chain fatty acids include caproic acid (hexanoic acid, C6:0), caprylic acid (octanoic acid, C8:0), capric acid (decanoic acid, C10:0) and lauric acid (dodecanoic acid, C12:0). Myristic acid (tetradecanoic acid, C14:0) can also be present in minor amounts. Medium-chain triglycerides most commonly used generally have a mixture of triglycerides of caprylic acid and capric acid, and contain 95% or greater of saturated fatty acids. The medium chain triglyceride component present in preferred compositions used in the methods of the present disclosure may consist of a homogeneous, single medium chain triglyceride compound type; more commonly, the medium chain triglyceride component is a mixture of two or more different medium chain triglyceride compounds.

The European Pharmacopoeia describes medium-chain triglycerides as the fixed oil extracted from the hard, dried fraction of the endosperm of *Cocos nucifera* L. (coconut) or from the dried endosperm of *Elaeis guineenis* Jacq. (African oil palm). The European Pharmacopoeia and the USPNF both have specifications for medium-chain triglycerides that require the presence of particular fatty acids is as follows: caproic acid (C6) ≤2.0%; caprylic acid(C8) 50.0-80.0%; capric acid (C10) 20.0-50.0%; lauric acid (C12) ≤3.0%; and myristic acid (C14) ≤1%.

Medium-chain triglycerides for use in preferred compositions comprise a mixture of triglycerides with fatty acid chains present in the following proportions: C6 ≤5%; C8 50-70%; C10 30-50%; and C12 ≤12%, for example C6 ≤0.5%; C8 55-65%; C10 35-45%; and C12 ≤1.5%.

Medium-chain triglycerides used in the preferred compositions may be derived from any known or otherwise suitable source.

Compositions used in the methods of the present disclosure may, advantageously, comprise one or more phospholipids. A particularly preferred phospholipid is phosphatidylcholine. The advantages brought about by phosphatidylcholine may be due, at least in part, to their amphipathic nature, e.g. due to properties as an emulsifier.

A particularly useful source of phospholipids, in particular phosphatidylcholine, is lecithin, and compositions used in the methods of the present disclosure advantageously comprise lecithin. Lecithin, when present in compositions, typically makes up at least 0.5% w/w of the composition, preferably at least 1% w/w of the composition. The lecithin preferably makes up 10% w/w or more of the composition, for example 20% w/w or more by weight, for example 30% w/w or more by weight of the composition. For example the lecithin may make up 0.5-80% w/w of the composition, for example 1-80% w/w, for example 20-80% w/w, for example 40-80% w/w, alternatively for example 0.5-75% w/w of the composition, for example, 1-40% w/w of the composition, for example 30-40% w/w of the composition, for example 30-35% w/w of the composition, for example, 30-75% w/w of the composition. Alternatively, the lecithin may make up 0.5-5% w/w of the composition, for example 1-5% w/w of the composition, for example 1-3% w/w of the composition, for example, 0.5-2% w/w of the composition, for example, 1-2% w/w of the composition. The weight ratio between the lecithin, when present, and the urolithin is generally in the range 0.02:1 to 3:1, for example, 0.03:1 to 1.2:1, for example 1:1 to 1.2:1, for example 1.1:1 to 1.2:1.

Commercially produced lecithin, which may be used in compositions described herein, typically contains the following major components: 33-35% soybean oil, 20-21% inositol phosphatides, 19-21% phosphatidylcholine, 8-20% phosphatidylethanolamine, 5-11% other phosphatides, 5% free carbohydrates, 2-5% sterols and 1% moisture.

Commercially produced lecithin, which may be used in compositions described herein, may for example be enriched with phosphatidylcholine, having a minimum of 5% w/w phosphatidylcholine in the lecithin, for example, having a minimum of 10% w/w phosphatidylcholine in the lecithin, for example, having a minimum of 15% w/w phosphatidylcholine in the lecithin, for example, having a minimum of 20% w/w phosphatidylcholine in the lecithin, for example, having a minimum of 25% w/w phosphatidylcholine in the lecithin, for example, having a minimum of 30% w/w phosphatidylcholine in the lecithin, for example, having a minimum of 32% w/w phosphatidylcholine in the lecithin, for example, having a minimum of 40% w/w phosphatidylcholine in the lecithin.

Lecithins may also be modified by one or more of the following processes to tailor their properties: alcohol extraction of particular phospholipids to produce a lecithin with a modified ratio of differing phospholipids; acetone extraction to remove oil, resulting in a powdered or granulated phospholipid blend; spray drying onto proteins as carriers; spray cooling with synthetic emulsifiers such as high melting mono- and di-glycerides to produce flaked or powdered products; modification by enzyme action (phospholipases, commonly in particular phospholipase A2), in particular partial hydrolysis to produce lecithins with pronounced emulsifying behaviour; hydrolysis of fatty acid groups by acids and alkali; acetylation; and hydroxylation of fatty acid chains and amino groups.

In some embodiments, the methods comprise administration of a composition comprising a compound of formula (I) or salt thereof, a medium chain triglyceride, and an emulsifier (e.g. lecithin).

Pharmaceutical compositions containing the compound of formula (I) or salt thereof may for example include additional pharmaceutically active compounds.

Additional components in a composition may be compounds that do not provide health benefits to the subject, but instead improve the composition in some other way, for example its taste, texture or shelf-life as mentioned above. The composition may thus further contain one or more compounds selected from emulsifiers, colorants, preservatives, gums, setting agents, thickeners, sweeteners and flavourings.

Suitable emulsifiers, stabilisers, colorants, preservatives, gums, setting agents and thickeners are well known in the art of manufacture of emulsions and other semi-liquids. Emulsifiers may include one or more of phosphatidylcholine, lecithin, polysorbates such as polysorbate 60 or polysorbate 80 (Tween-60 and Tween-80), and glycerol monostearate (GMS). Glycerol monostearate is also known as glyceryl monostearate.

Stabilisers may be used in a composition described herein. Many compositions are stable suspensions without the need for an added stabiliser. A stable suspension is one that does not undergo a phase separation over time. For certain compositions, the stability can be improved by inclusion of an added stabiliser. Suitable stabilisers for use in compositions of the invention include glycerol monostearate (GMS), silicon dioxide and vegetable shortening. An exemplary stabiliser is GMS and preferred compositions of the invention contain GMS. Its properties also make GMS a good solvent for phospholipids, such as found in lecithin for example. GMS exists in two polymorphs: the α-form is dispersible and foamy, useful as an emulsifying agent or preservative. The β-form is suitable for wax matrices. The α-form is converted to the β-form when heated at 50°C.

GMS falls into two distinct grades: 40-55 percent monoglycerides, and 90 percent monoglycerides. 40-55 percent monoglycerides as defined by the European Pharmacopoeia describes GMS as a mixture of monoacylglycerols, mostly monostearoylglycerol, together with a quantity of di- and tri-glycerols. In particular, the 40-55 grade contains 40-55% monoacylglycerols, 30-45% diacylglycerols, and 5-15% of triacylglycerols. The 99 percent grade contains not less than 90% of monoglycerides. The monoglycerides in commercial GMS products are mixtures of variable proportions of glyceryl monostearate and glyceryl monopalmitate. The European Pharmacopoeia further divides glyceryl monostearate 40-55 into three types according to the proportion of stearic ester in the mixture. Type 1 contains 40.0-60.0% stearic acid, and the sum of palmitic and stearic acids is ≤90%. Type 2 contains 60.0-80.0% stearic acid, and the sum of palmitic and stearic acids is ≤90%. Type 3 contains 90.0-99.0% stearic acid, and the sum of palmitic and stearic acids is ≤96%. Any form of GMS may be used in the compositions.

In some embodiments, the method comprises administration of a composition comprising a medium chain triglyceride, the compound of formula (I) or a salt thereof (e.g.urolithin A), and a stabiliser, for example glycerol monostearate. In some embodiments the method involves administration of a composition comprising an emulsifier and a stabiliser.

*Met al* chelators or sequestrants such as sodium calcium salts of ethylenediamine tetra acetic acid (EDTA) may also be used. Other components that may be included in formulations of the invention include polyethylene glycols, silicon dioxide, vegetable shortening and beeswax.

A flavouring may be beneficial in compositions used in the methods described herein. In a liquid or semi-liquid composition, fruit flavour can be provided for example by inclusion of a fruit sauce or puree. Typical flavourings include strawberry, raspberry, blueberry, apricot, pomegranate, peach, pineapple, lemon, orange and apple. Generally, fruit flavourings include fruit extract, fruit preserve or fruit puree, with any of a combination of sweeteners, starch, stabilizer, natural and/or artificial flavours, colourings, preservatives, water and citric acid or other suitable acid to control the pH.

A unit dose composition used in the methods described herein preferably contains 250mg or 500mg of the compound of formula (I), for example 250mg or 500mg of urolithin A. A unit dose may for example be in the form of a tablet or capsule, in the form of a drink provided in a container such as a bottle or pouch sufficient to hold a single dose (e.g. 50 to 500ml, 100 to 300 ml, for example, 250ml or 500ml). In a further alternative example, which is preferred, the unit dose is in the form of a softgel capsule, e.g. containing 250mg of urolithin A.

A representative urolithin composition is shown in the Table below:

### Representative composition A:

| | |
|---|---|
| Composition | Per 100 g |
| Medium Chain Triglycerides | 10-85 g |
| Urolithin A | 10-50 g |
| Lecithin (comprising minimum phosphatidylcholine content of 32%w/w) | 10-50 g |
| Glycerol Monostearate | 0-5g |

### Immunotherapy treatment compositions

Provided herein are compositions comprising an immunotherapy treatment, for example, an antibody or a fusion protein for use in a combination of the invention. Also provided herein are compositions comprising an anti-PD-1 antibody, an anti-PD-L1 antibody, an anti-CTLA4 antibody or a fusion protein. The compositions include bulk drug compositions useful in the manufacture of pharmaceutical compositions (e.g., impure or non-sterile compositions) and pharmaceutical compositions (i.e., compositions that are suitable for administration to a subject or patient) which can be used in the preparation of unit dosage forms. The compositions (e.g. pharmaceutical compositions) comprise an effective amount of an immunotherapy treatment, such as an anti-PD-1 antibody, an anti-PD-L1 antibody, an anti-CTLA4 antibody or a fusion protein and a pharmaceutically acceptable carrier. In specific embodiments, the compositions (e.g., pharmaceutical compositions) comprise an effective amount of one or more antibodies or proteins, such as an anti-PD-1 antibody, an anti-PD-L1 antibody, an anti-CTLA4 antibody or a fusion protein.

Pharmaceutical compositions may be formulated in any conventional manner using one or more pharmaceutically acceptable carriers, such as adjuvants, or excipients.

Adjuvants include, but are not limited to, Freund's adjuvant (complete and incomplete) or MF59C.1 adjuvant.

Suitable pharmaceutical carriers include sterile liquids, such as water and oils, including those of petroleum, animal, vegetable or synthetic origin, such as peanut oil, soybean oil, mineral oil, sesame oil and the like. In one embodiment, water is a carrier when the pharmaceutical composition is administered intravenously. Saline solutions and aqueous dextrose and glycerol solutions can also be employed as liquid carriers, particularly for injectable solutions.

Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

In a specific embodiment, an immunotherapy treatment for use in a combination of the invention is administered to a subject in accordance with the methods described herein and is administered as a pharmaceutical composition.

Generally, the components of the pharmaceutical compositions comprising an immunotherapy treatment, such as an anti-PD-1 antibody, an anti-PD-L1 antibody, an anti-CTLA4 antibody or a fusion protein are supplied either separately or mixed together in unit dosage form, for example, as a dry lyophilized powder or water free concentrate in a hermetically sealed container such as an ampoule or sachette indicating the quantity of active agent. Where the immunotherapy treatments, such as an anti-PD-1 antibody, an anti-PD-L1 antibody, an anti-CTLA4 antibody or a fusion protein is to be administered by infusion, it can be dispensed with an infusion bottle containing sterile pharmaceutical grade water or saline (e.g., PBS). Where the immunotherapy treatment, such as an anti-PD-1 antibody, an anti-PD-L1 antibody, an anti-CTLA4 antibody or a fusion protein, is administered by injection, an ampoule of sterile water for injection or saline can be provided so that the ingredients may be mixed prior to administration.

In some embodiments, the immunotherapy treatment, such as an anti-PD-1 antibody, an anti-PD-L1 antibody, an anti-CTLA4 antibody or a fusion protein, may be formulated for administration by any method known to one of skill in the art, including but not limited to, parenteral (e.g., subcutaneous, intravenous, intra-tumoural or intramuscular) administration. In one embodiment, the immunotherapy treatment, such as an anti-PD-1 antibody, an anti-PD-L1 antibody, an anti-CTLA4 antibody or a fusion protein, is formulated for local or systemic parenteral administration, for example intra-tumoural administration. In a specific embodiment, the immunotherapy treatment, such as an anti-PD-1 antibody, an anti-PD-L1 antibody, an anti-CTLA4 antibody or a fusion protein, is formulated for subcutaneous or intravenous administration, respectively. In one embodiment, the immunotherapy treatment, such as an anti-PD-1 antibody, an anti-PD-L1 antibody, an anti-CTLA4 antibody or a fusion protein, is formulated in a pharmaceutically compatible solution.

The immunotherapy treatment, such as an anti-PD-1 antibody, an anti-PD-L1 antibody, an anti-CTLA4 antibody or a fusion protein, can be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

For the avoidance of doubt, combinations of the invention may be formulated in the same composition or formulated in separate compositions for simultaneous, separate or sequential administration.

Also within the scope of the present invention are kits, including pharmaceutical kits, comprising a combination of the invention for therapeutic uses. Kits typically include a label indicating the intended use of the contents of the kit and instructions for use. The term "label" includes any writing, or recorded material supplied on or with the kit, or which otherwise accompanies the kit. Certain embodiments of a pharmaceutical kit, comprise a urolithin, for example, urolithin A, and an immunotherapy treatment, such as an immune checkpoint blockage therapy in unit dosage form.

In one embodiment there is provided, a kit for the treatment of a disease state associated with inhibition of T-cell activation, comprising:
(a) a urolithin;
(b) an immunotherapy treatment, for example, an immune checkpoint blockage therapy;
(c) a container, or containers, for containing said agents; and
(d) optionally instructions for simultaneous, separate or sequential administration.

The term 'antibody or functional part thereof' is used in the broadest sense. It may be man-made such as monoclonal antibodies (mAbs) produced by conventional hybridoma technology, recombinant technology and/or a functional fragment thereof. It may include both intact immunoglobulin molecules for example a polyclonal antibody, a monoclonal antibody (mAb), a monospecific antibody, a bispecific antibody, a polyspecific antibody, a human antibody, a humanized antibody, an animal antibody (e.g. camelid antibody), chimeric antibodies, as well as portions, fragments, regions, peptides and derivatives thereof (provided by any known technique, such as, but not limited to, enzymatic cleavage, peptide synthesis, or recombinant techniques), such as, for example, mmunoglobulin devoid of light chains, Fab, Fab', F (ab')2, Fv, scFv, antibody fragment, diabody, Fd, CDR regions, or any portion or peptide sequence of the antibody that is capable of binding antigen or epitope. In one embodiment, the functional part is a single chain antibody, a single chain variable fragment (scFv), a Fab fragment, or a F(ab')2 fragment.

An antibody or functional part is said to be "capable of binding" a molecule if it is capable of specifically reacting with the molecule to thereby bind the molecule to the antibody. Antibody fragments or portions may lack the Fc fragment of intact antibody, clear more rapidly from the circulation, and may have less non-specific tissue binding than an intact antibody. Examples of antibody may be produced from intact antibodies using methods well known in the art, for example by proteolytic cleavage with enzymes such as papain (to produce Fab fragments) or pepsin (to produce F(ab')2 fragments). Portions of antibodies may be made by any of the above methods, or may be made by expressing a portion of the recombinant molecule. For example, the CDR region(s) of a recombinant antibody may be isolated and sub-cloned into an appropriate expression vector.

In one embodiment, an antibody or functional part is a human antibody. The use of human antibodies for human therapy may diminish the chance of side effects due to an immunological reaction in a human individual against nonhuman sequences. In another embodiment, the antibody or functional part is humanized. In another embodiment, an antibody or functional part is a chimeric antibody. This way, sequences of interest, such as for instance a binding site of interest, can be included into an antibody or functional part.

In one embodiment, the antibody may have an IgG, IgA, IgM, or IgE isotype. In one embodiment, the antibody is an IgG.

The term "cancer" refers to a disease characterized by the rapid and uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukaemia, lung cancer and the like. The terms "tumour" and "cancer" are used interchangeably herein, e.g., both terms encompass solid and liquid, e.g., diffuse or circulating, tumours. As used herein, the term "cancer" or "tumour" includes premalignant, as well as malignant cancers and tumours.

The term "carrier" refers to a diluent, adjuvant, excipient, or vehicle with which the therapeutic is administered.

The term 'excipient' refers to a substance formulated alongside the active ingredient of a medication, included, for example, for the purpose of long-term stabilization, bulking up solid formulations that contain potent active ingredients in small amounts (thus often referred to as "bulking agents", "fillers", or "diluents"), or to confer a therapeutic enhancement on the active ingredient in the final dosage form, such as facilitating drug absorption, reducing viscosity or enhancing solubility.

The abbreviation 'HumAbs' refers to humanized monoclonal antibodies.

The term 'lecithin' designates any group of fatty substances occurring in animal and plant tissues including phosphoric acid, choline, fatty acids, glycerol, glycolipids, triglycerides, and phospholipids (e.g., phosphatidylcholine, phosphatidylethanolamine, and phosphatidylinositol). Commercial lecithin obtained from soya and sunflower comprises the phospholipids phosphatidylcholine, phosphatidylinositol, phosphatidylethanolamine, and phosphatidic acid. Lecithin may be obtained by chemical extraction from its source in a nonpolar solvent such as hexane, ethanol, acetone, petroleum ether or benzene, or by mechanical extraction. In particular, lecithin may be obtained by extraction from sources including soybeans, eggs, milk, rapeseed, cottonseed and sunflower. Commercial lecithin for use in edible formulations may be readily purchased.

The term 'immune checkpoint blockage therapy' or 'immune checkpoint blockage therapies' relates to therapeutic approaches which remove inhibitory signals of T-cell activation which enable reactive T-cells to overcome regulatory mechanisms and mount an effective immune response, for example, tumour-reactive T cells to mount an effective antitumour response. For a review of checkpoint blockage therapy see: Wei et al (2018) Cancer Discovery 8(8), 1-18. Examples of immune checkpoints include: PD-1, CTLA-4, lymphocyte activation gene-3 (LAG-3), T-cell immunoglobulin and ITIM domain (TIGIT), and T-cell immunoglobulin-3 (TIM-3).

The term 'immunotherapy treatment' refers to any treatment whose mechanism of action, in part or predominantly, acts via enhancing an individual's immune response.

The abbreviation 'mAbs' refers to monoclonal antibodies.

The term 'PD-1 antagonist refers to any agent which blocks the inhibitory effect of PD-1 on the immune system. For example, a PD-1 antagonist includes agents, which directly block the binding of PD-1 to its receptor, and agents, which have an allosteric effect on the activity of PD-1.

The term "pharmaceutically acceptable" means approved by a regulatory agency of the Federal or a state government or listed in the U.S. Pharmacopeia or other generally recognized pharmacopeia for use in animals, and more particularly in humans.

The term "Programmed Death-1 (PD-1)" refers to an immunoinhibitory receptor belonging to the CD28 family. PD-1 is expressed predominantly on previously activated T cells in vivo, and binds to two ligands, PD-L1 and PD-L2. The term "PD-1" as used herein includes human PD-1 (hPD-1), variants, isoforms, and species homologs of hPD-1, and analogues having at least one common epitope with hPD-1. The complete hPD-1 sequence can be found under GenBank Accession No. U64863.

The term "Programmed Death Ligand-1 (PD-L1)" is one of two cell surface glycoprotein ligands for PD-1 (the other being PD-L2) that down-regulate T cell activation and cytokine secretion upon binding to PD-1. The term "PD-L1" as used herein includes human PD-L1 (hPD-L1), variants, isoforms, and species homologs of hPD-L1, and analogues having at least one common epitope with hPD-L1. The complete hPD-L1 sequence can be found under GenBank Accession No. Q9NZQ7.

The term "Programmed Death Ligand-2 (PD-L2)" is one of two cell surface glycoprotein ligands for PD-1 (the other being PD-L2) that down-regulate T cell activation and cytokine secretion upon binding to PD-1. The term "PD-L2" as used herein includes human PD-L2 (hPD-L2), variants, isoforms, and species homologs of hPD-L2, and analogs having at least one common epitope with hPD-L2. The complete hPD-L1 sequence can be found under GenBank Accession No. Q9BQ51.

The term, "separate" administration means the administration of each of two or more compounds to a patient from non-fixed dose dosage forms simultaneously, substantially concurrently, or sequentially in any order. There may, or may not, be a specified time interval for administration of each the compounds.

The term "sequential" administration means the administration of each of two or more compounds to a patient from non-fixed (separate) dosage forms in separate actions. The administration actions may, or may not, be linked by a specified time interval. For example, administering compounds over a specified time such as once every 14 to 21 days..

The term "simultaneous" administration means the administration of each of two or more compounds to a patient in a single action such as where each compound are administered independently at substantially the same time or separately within time intervals that allow the compounds to show' a cooperative therapeutic effect.
The invention will now be illustrated with respect to the following non-limiting examples

### Summary of the Figures

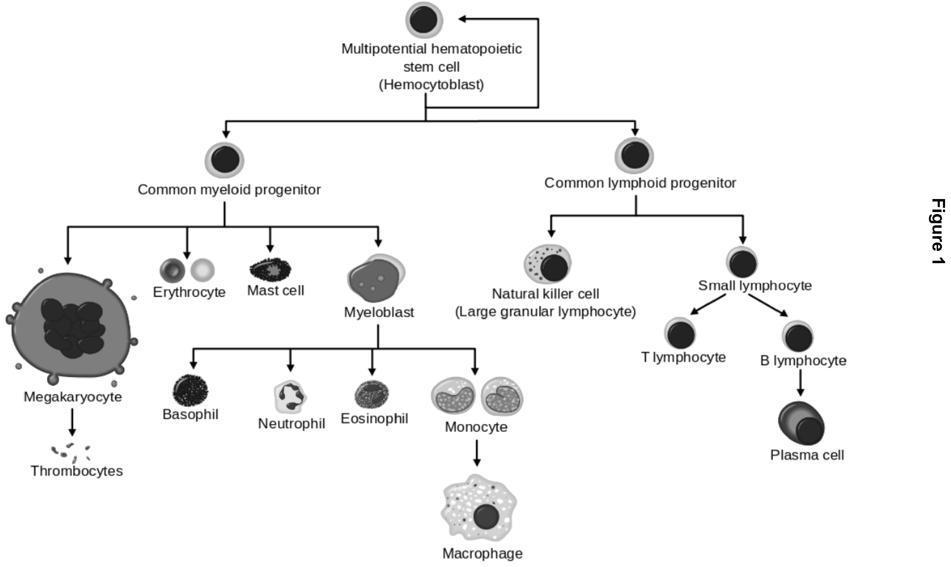

- Figure 1:: Blood cell lineages that originate from hematopoietic stem cells.
- Figure 2:: AOM model of colorectal cancer. Representative images of H&E staining of colonic mucosa collected in control and UA 200 mpk (corresponding to 2.28g of UA per kg of diet) treated animals. Below are the graph showing the quantification of the number of lesions (left) and their average size (right) in control, Urolithin A (UA) 50mpk (UA LD) and UA 200mpk (UA HD) treated animals.
- Figure 3.: AOM model of colorectal cancer. Representative images of anti-CD3 immunostaining of colonic mucosa collected in control and UA 200 mpk (corresponding to 2.28g of UA per kg of diet) treated animals. Below are the graph showing the quantification of the number of CD3 positive cells (left) and percentage of CD3 positive cells (right) in control and UA 200mpk (UA HD) treated animals.
- Figure 4.: APTK organoid model. Tumour volume of subcutaneously injected APTK organoid xenograft tumours in animals fed a control or UA 200 mpk diet.
- Figure 5.: APTK organoid model. Quantification of the number of CD3 positive cells (left) and percentage of CD3 positive cells (right) in control (top) and UA 200mpk (Urolithin A) treated animals.
- Figure 6.: APTK organoid model. Representative images of anti-cleaved caspase 3 immunostaining of APTK organoid derived tumours collected in control and UA 200 mpk treated animals. Below is the graph showing the quantification of the percentage of cleaved caspase 3 positive cells in control and UA 200mpk (Urolithin A) treated animals.
- Figure 7.: APTK organoid model. Tumour volume of subcutaneously injected APTK organoid xenograft tumours in animals fed a control diet, UA 200 mpk diet combined with anti-CD8 injections or its isotype q2d.
- Figure 8.: APTK organoid model. Tumour volume of subcutaneously injected APTK organoid xenograft tumours in animals fed a control diet, UA 200 mpk diet combined with anti-PD1 injections or its isotype q3d. *P<0.05; **P<0.01 (Mann-Whithney-Test for tumour size at end of experiment).

### EXAMPLES

The invention will now be illustrated with respect to the following non-limiting examples

### Example 1: Urolithin A (UA) reduces lesion incidence and size, and increases the infiltration of T cytotoxic cells inside tumours in a mouse model of colorectal cancer (AOM model)

A model of sporadic tumourigenesis can be obtained applying repetitively azoxymethane (AOM), without dextran sodium sulphate, to female wild-type FVB mice. In particular, tumours are induced by six weekly intraperitoneal injections of AOM (10mg/kg) in wild type animals. Mice are analysed 18-24 weeks later. Urolithin A is applied in the diet at a dose of 50 or 200mg/kg mouse a day (mpk), or control lab diet one week before starting the 6 injections of AOM, during the 6 weeks of injections with AOM, and for approximately 20 weeks following these injections, for a total of ca. 27-30 weeks. At the end of the treatment period, mice are euthanized by cervical dislocation for the collection of intestinal tissues. Colonic tissue is stained for hematoxylin and eosin in order to count and measure the number of lesions.

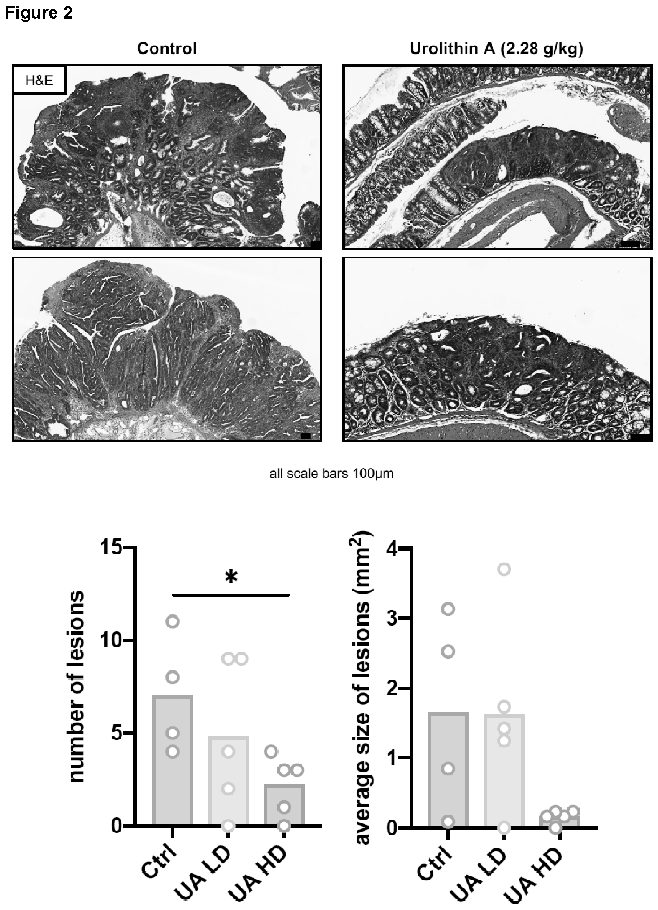

As can be seen on Figure 2, treatment of animals with UA at 200 mpk significantly decreases the number of lesions in the AOM model of colorectal cancer. The lesions in UA treated animals also appear to be of smaller size.

In order to determine whether this effect can be explained at least in part by an immunogenic effect, colonic mucosa is also tested for CD3 positive cells, a marker of the T cell lineage.

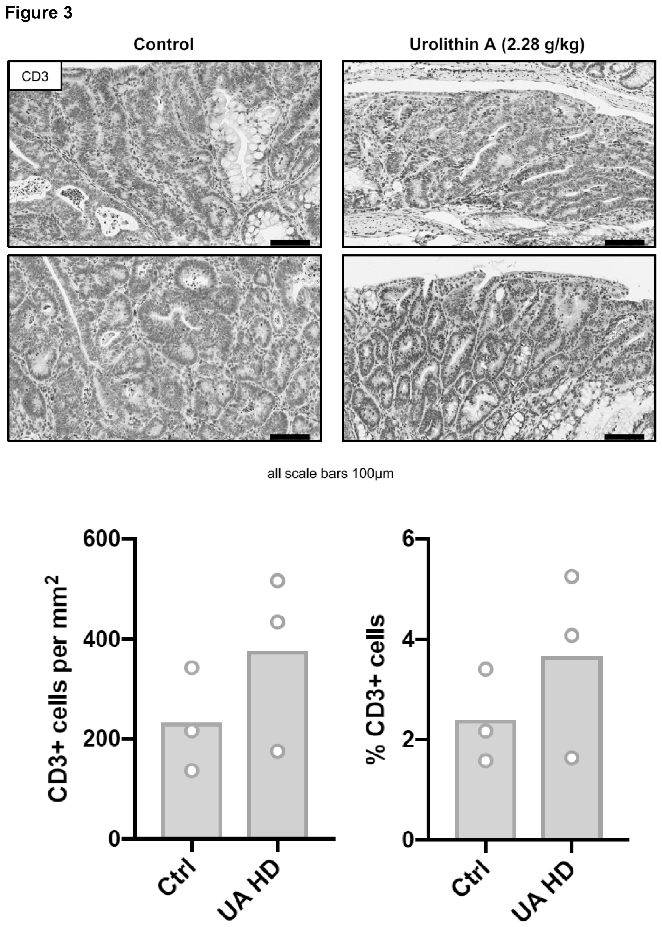

Figure 3 shows the results of the anti-CD3 immunostaining. Colonic mucosa of animals treated with UA at 200 mpk tend to have more CD3 positive cells, providing evidence of an active recruitment of T cells for the elimination of cancer cells.

### Example 2: Urolithin A (UA) inhibits growth of APTK subcutaneous xenograft tumours and increases the infiltration of T cytotoxic cells inside tumours

Colorectal cancer organoids (from here on also referred to as APTK organoids) are generated by the selective introduction of tumourigenic mutation in the gene APC (A), p53 (P), Tgf-beta1 (T) and K-ras (K). Since APTK organoids have been developed on a C57/BL6 genetic background, they can be transplanted to generate tumours in wild type BL6 mice with an intact immune system that allows to study the immune response towards the developing tumour. One week prior to subcutaneous transplantation, the mice are fed either a Urolithin A-containing or control diet. Tumour growth is assessed by calliper measurements. The resulting tumours are analysed histologically.

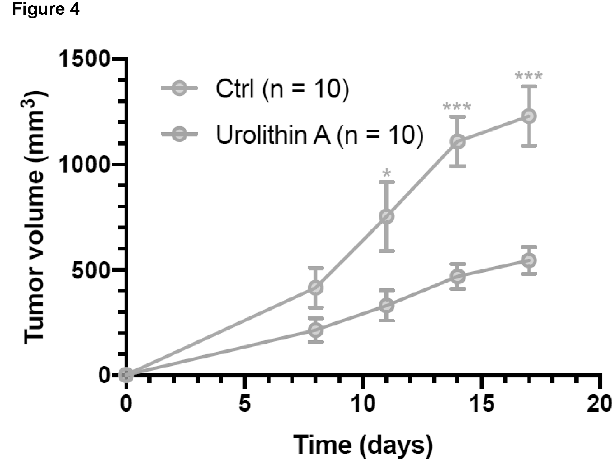

As is represented in Figure 4, animals treated with UA 200 mpk present a significant lower tumour volume compared to animals who received a control diet, meaning that UA prevents tumour growth.

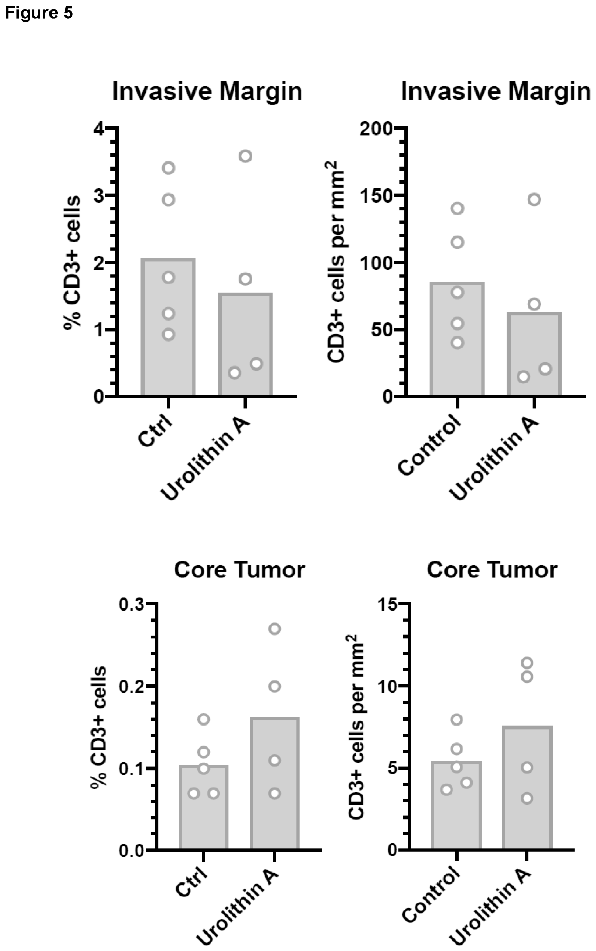

In order to determine whether this effect can be explained at least in part by an immunogenic effect, APTK-derived subcutaneous tumours are also tested for CD3 positive cells, a marker of the T cell lineage. Figure 5 shows the results of the anti-CD3 immunostaining. Tumours of animals treated with UA at 200 mpk tend to have less CD3 positive cells at the invasive margin, but more CD3 positive cells inside the core of the tumours, providing evidence of an active recruitment of T cells for the elimination of cancer cells.

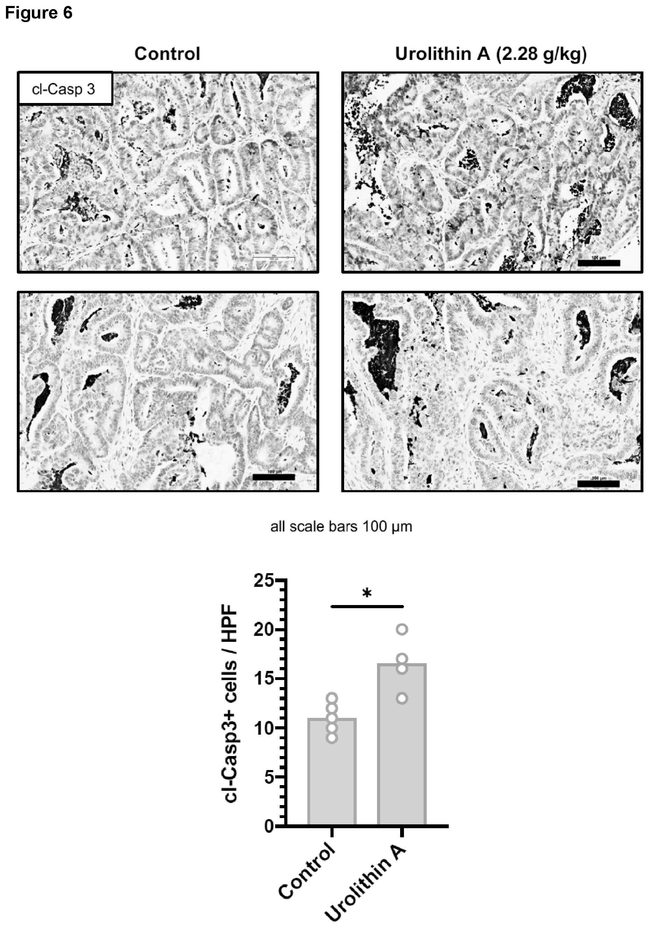

Figure 6 shows the staining of the tumours for cleaved caspase 3, a marker of apoptosis. UA treatment at 200 mpk led to a significant increase of cleaved Casp3 staining, meaning that it induces apoptosis in APTK organoid derived tumour cells.

### Example 3: Urolithin A (UA) requires the presence of CD8+ lymphocytes to promote its anti-tumourigenic effect

To prove that the expected tumour-suppressive inflammatory effect of Urolithin A is due to a cytotoxic T cell response, Urolithin A treatment is combined with abrogation of CD8+ cytotoxic T cells. For this model, C57/BL6 were maintained on a Urolithin A 200mpk or control diet. One week after starting the dietary intervention, mice were transplanted subcutaneously with APTK organoids as described above. Starting at day 3, these mice received 5 intraperitoneal injections (150µg every second day) of an anti-CD8 or isotype control antibody (BioXCell) to deplete CD8+ cytotoxic T cells. As in the previous experiments, tumour growth is measured by calliper measurements.

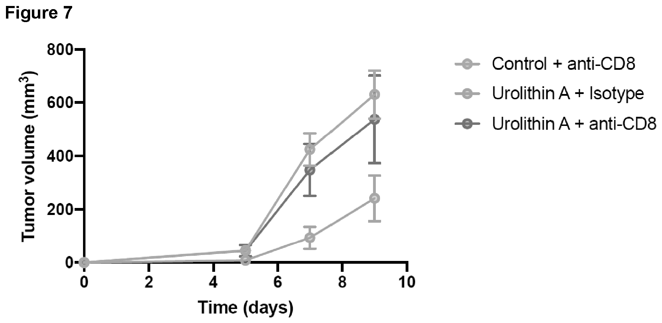

Figure 7 shows that the anti-tumoural effect of UA is lost when the CD8+ cytotoxic T cells are abrogated using an anti-CD8 treatment, meaning that at least part of the effect of UA for treating tumours is driven by an immunogenic effect.

### Example 4: Urolithin A (UA) acts in synergy with anti-PD1 immunotherapy

C57BL/6 mice are transplanted subcutaneously with APTK organoids as described in Example 2 and either, fed with a Urolithin A 200 mpk or control diet (initiated one week prior to subcutaneous injection) and receive 4 intraperitoneal injections (200µg every third day, starting day 5) of a blocking anti-PD1 or isotype control antibody (BioXCell). As in the previous experiments, tumour growth is measured by calliper measurements.

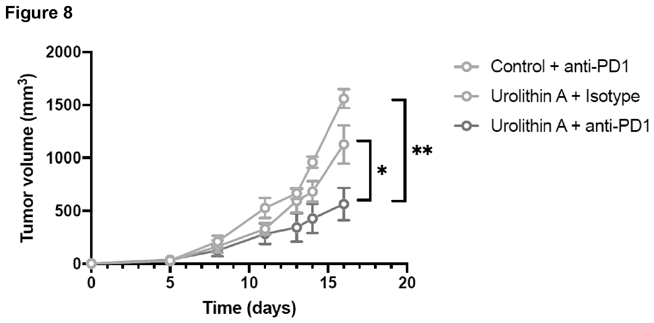

Figure 8 shows that no difference was observed in the effect of Urolithin A (200 mpk) alone (i.e. with the isotype control antibody) versus the anti-PD1 antibody treatment with regards to tumour growth. However, when anti-PD-1 and Urolithin A is co-administered we observe a dramatic synergistic effect on the slowing of the tumour growth, providing clear evidence that Urolithin A potentiates a much stronger biological anti-tumour response to anti-PD1 therapies than anti-PD1 therapies alone

### Equivalents

The invention has been described broadly and generically herein. Those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention. Further, each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

### Incorporation by Reference

The contents of the articles, patents, and patent applications, and all other documents and electronically available information mentioned or cited herein, are hereby incorporated by reference in their entirety to the same extent as if each individual publication was specifically and individually indicated to be incorporated by reference. Applicants reserve the right physically to incorporate into this application any and all materials and information from any such articles, patents, patent applications, or other physical and electronic documents.

## Claims

1. A combination of a urolithin, for example, urolithin A, and an immunotherapy treatment for the treatment of a disease selected from cancer and infectious diseases wherein the immunotherapy treatment is CAR-T cell immunotherapy (chimeric antigen receptor T-cell therapy).

2. A combination as claimed in Claim 1 wherein the disease is cancer.

3. A combination as claimed in Claim 2 wherein the cancer is selected from: bladder cancer melanoma, including paediatric melanoma, lung cancer, such as small cell lung cancer, non- small cell lung cancer, squamous cell lung carcinoma, head and neck cancer, such a head and neck squamous cell carcinoma, B-cell lymphoma, such as Hodgkin's lymphoma, T-cell lymphoma, urothelial cancer, renal cancer, hepatocellular cancer, skin cancer, such as merkel cell carcinoma, gastric cancer and gastroesophageal cancer.

4. A combination as claimed in Claim 2 wherein the cancer is selected from: a microsatellite instability high (MSI-H) or mismatch repair deficient (dMMR) solid tumour.

5. A combination as claimed in Claim 2 wherein the cancer is colorectal cancer.

6. A combination as claimed in Claim 1 wherein the disease is an infectious disease.

7. A combination as claimed in Claim 6 wherein the infectious disease is selected from viral, bacterial, fungal and parasitic infections.

8. A combination as claimed in Claim 7 where the viral Infection is selected from lymphocytic choriomeningitis virus (LCMV), HIV, hepatitis B virus (HBV), or hepatitis C virus (HCV).

9. A combination as claimed in Claim 7 wherein the bacterial infection is selected from Helicobacter pylori, Mycobacterium tuberculosis (MTB), sepsis (gram positive bacteria such as staphylococcus) and nosocomial (hospital acquired infections, such as C. difficile).

10. A combination as claimed in Claim 7 wherein the fungal infection is selected from Candidiasis, Aspergillosis and Cryptococci.

11. A combination as claimed in Claim 7 wherein the parasitic infection is selected from helminth parasites, including Schistosoma mansoni, Schistosoma japonicum, Schistosoma haematobium, Fasciola hepatica, and Heligmosomoides polygyrus, Leishmania such as Leishmania donovani, Leishmania chagasi and Leishmania Mexicana, Plasmodium, such as Plasmodium berghei and Plasmodium falciparum, Toxoplasma, such as Toxoplasma gondi.

12. A combination as claimed in any one of the preceding claims further comprising one of further therapies, for example, radiation therapy and/or one or more additional therapeutic agents.

13. A combination as claimed in claim 12 wherein the one or more therapeutic agents are selected from anti-cancer agents, anti-viral agents, anti-inflammatory agents, and or adjuvants

14. A pharmaceutical composition comprising a combination of a urolithin, for example, urolithin A, and an immunotherapy treatment, wherein the immunotherapy treatment is CAR-T cell immunotherapy (chimeric antigen receptor T-cell therapy)..

15. A kit for the treatment of a disease selected from cancer and infectious diseases, comprising:
(a) a urolithin, for example, urolithin A;
(b) CAR-T cells;
(c) a container, or containers, for containing said agents; and
(d) optionally instructions for simultaneous, separate or sequential administration.
